# EUROPÄISCHE PATENTANMELDUNG

(11) **EP 0 677 279 A1**
(43) Veröffentlichungstag der Anmeldung: **18.10.1995**
(21) Anmeldenummer: 95104867.7
(22) Anmeldetag: 31.03.1995
(51) Int. Cl.: A61C 5/06

(54) **Vorratsspritze für Dentalmasse**

(30) Priorität: 15.04.1994 DE 9406342 U
(71) Anmelder: Mühlbauer, Ernst, D-22547 Hamburg (DE)
(72) Erfinder: Mühlbauer, Ernst, D-22547 Hamburg (DE)
(74) Vertreter: Glawe, Delfs, Moll & Partner

(57) **Zusammenfassung**

Vorratsspritze für Dentalmasse, insbesondere für Füllungen und Verblendungen, deren Kolben durch eine Schraubspindel (5) verschiebbar ist, die eine Handhabe aufweist. Diese ist erfindungsgemäß als Farbmuster für die in der Vorratsspritze enthaltene Dentalmasse ausgebildet. Damit auch über ein schmales Farbmuster eine hinreichende Drehkraft auf die Spindel aufgebracht werden kann, ist der Spritzenkörper zur Herabsetzung der erforderlichen Drehkraft zweckmäßigerweise verengungsfrei ausgebildet.

## Beschreibung

Die Erfindung betrifft eine Vorratsspritze für Dentalmasse, deren Kolben durch eine Schraubspindel verschiebbar ist, die mittels einer Handhabe verdrehbar ist.

Um die Farbe von Füll- oder Verblendmasse möglichst genau an die Farbe der Zähne des Patienten anpassen zu können, verfügt der Zahnarzt über eine Reihe entsprechend farblich abgestimmter Dentalmassen und zugehörige Farbmuster, die beispielsweise fächerartig in einem sog. Zahnfarbenring vereinigt sind. Die einzelnen Muster sind schmale, keilförmig flach zulaufende Plättchen, deren Gestalt einem Schneidezahn nicht unähnlich ist. Sie sind an dem Zahnfarbenring derart vereinzelbar, daß sie gesondert von den übrigen Mustern zum Vergleich an die Zähne angehalten werden können. Sie tragen Buchstaben- oder Nummernbezeichnungen, die es gestatten, ihnen diejenige Vorratsspritze zuzuordnen, die die entsprechend farblich abgestimmte Dentalmasse enthält. Bei dieser Zuordnung sind Fehler möglich. Die Erfindung sucht deshalb nach einer Möglichkeit, die Zuordnung von Muster und Vorratsspritze zu vereinfachen.

Die erfindungsgemäße Lösung besteht darin, daß die Handhabe der Schraubspindel der Vorratsspritze ein Farbmuster für die in der Vorratsspritze enthaltene Dentalmasse umfaßt. Der Zahnarzt benutzt die Handhabe, also das hintere Ende der Vorratsspritze, als Farbmuster. Sobald er das richtige Muster gefunden hat, hält er auch schon die richtige Vorratsspritze in der Hand. Die Zuordnung über Farbcodes wird ihm erspart und Zuordnungsfehler sind dadurch ausgeschlossen. Wurde von ihm oder seiner Helferin eine Auswahl getroffen, so kann er diese, wenn er die Vorratsspritze in der Hand hat, noch einmal direkt nachprüfen.

Zwar ist es möglich, das Muster in der Form bekannter Handhaben zum Drehen der Spindel auszubilden, die beispielsweise als Flügelpaar ausgebildet sind; angenehmer für den Vergleich mit der Zahnfarbe ist es aber, wenn das Muster nach hinten etwa in der Längsrichtung der Spindel an diese anschließt.

Damit man trotz der Schmalheit des Musters die Spindel selbst dann leicht drehen kann, wenn dafür ein beträchtliches Drehmoment erforderlich ist, können zusätzliche Handhabungsteile vorgesehen sein. Zweckmäßiger ist es aber, wenn keine oder nur kleine weitere Teile einer Handhabe vorgesehen werden und die Spindel allein mittels des Farbmusters gedreht werden kann. Das ist aber nur dann möglich, wenn die von der Spindel geforderte Drehkraft gering ist. Dies wird erfindungsgemäß dadurch erreicht, daß der Spritzenkörper verengungsfrei in die Ausbringöffnung mündet. Damit läßt sich die Spindel sogar trotz der Schmalheit des Farbmusters einhändig verdrehen. Eine verengungsfreie Ausbringöffnung ist bislang nur bei körnigen Substanzen wie Amalgam oder Knochenspäne bekannt (US-A 4,801,263, DE-B 27 41 185).

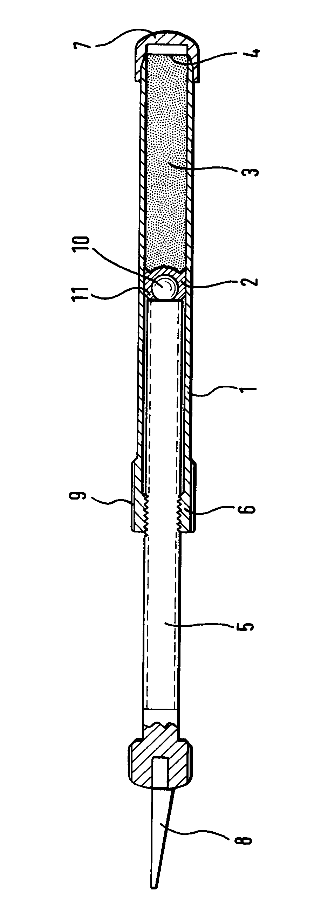

Die Erfindung wird im folgenden näher unter Bezugnahme auf die Zeichnung erläutert, die ein vorteilhaftes Ausführungsbeispiel in einem Längsschnitt veranschaulicht.

Der Spritzenkörper 1 enthält einen Kolben 2, der zum Ausbringen der zähen Dentalmasse 3 aus der Ausbringöffnung 4 des Spritzenkörpers 1 mittels einer Gewindespindel 5 verschiebbar ist, die in dem Mutterteil 6 des Spritzenkörpers 1 verschraubbar ist. Die Ausbringöffnung 4 des Spritzenkörpers 1 kann mittels eines Deckels 7 verschlossen werden.

Am hinteren Ende ist die Spindel 5 mit einem Farbmuster 8 drehfest verbunden, das die für derartige Farbmuster bekannte, schneidezahnähnliche Gestalt hat; d.h. eine Breite von beispielsweise 7 mm und eine Länge von beispielsweise 10 mm besitzt, wobei er zu seinem Ende hin flach keilförmig zuläuft, nämlich mit einer Dicke zwischen etwa 3 mm an seinem vorderen und etwa 0,5 mm an seinem freien Ende. Selbstverständlich könnten diese Maße auch anders gewählt werden, wenn dies für die Muster- oder Handhabungszwecke vorteilhaft erscheint. Jedoch hat sich gezeigt, daß selbst bei Ausbildung in dieser traditionellen Form der Musterkörper als Handhabe zum Verdrehen der Spindel 5 jedenfalls dann ausreicht, wenn der Spritzenkörper 1 bis zu seiner Ausbringöffnung 4 hin verengungsfrei ausgebildet ist. Dann ist nämlich nur ein geringes Drehmoment an der Spindel 5 zum Ausbringen der zähen Dentalmasse erforderlich.

Handhabungsflügel an der Spindel sind in diesem Fall entbehrlich und brauchen auch nicht am Spritzenkörper vorgesehen zu sein. Es genügt eine Längsriefelung 9 am Spritzenkörper. Gegebenenfalls kann außerdem ein zwischen der Spindel 5 und dem Muster 8 angeordneter Drehknopf mit einer Längsriefelung versehen sein. Da die Spritze wegen des Fehlens der ausladenden Handhabungsflügel einen geringeren seitlichen Platzbedarf hat, kann sie raumsparender verpackt werden als herkömmtliche. Dieser Vorteil ist unabhängig von der Ausführung der Spindelhandhabe als Farbmuster. Die Kombination der verengungsfreien Ausführung des Spritzenkörpers mit dem Fehlen von seitlich ausladenden Handhaben am Spritzenkörper und an der Spindel verdient daher ggf. Schutz unabhängig von der Ausführung der Spindelhandhabe als Farbmuster.

Am vorderen Ende der Spindel 5 ist diese fest mit einem kugelförmig ausgebildeten Kupplungsteil 10 versehen, das in eine entsprechende Kupplungshöhlung des Kolbens 2 eingeschnappt werden kann, wenn sich der Kolben außerhalb des Spritzenkörpers befindet. Mit einem verengten Querschnittsteil 11 greift der Kolben hinter den kugelförmigen Kupplungsteil 10. Wenn sich der Kolben innerhalb des Spritzenkörpers 1 befindet, ist der verengte, hintere Teil des Kolbens an elastischer Aufweitung gehindert. Dadurch sind Spindel 5 und Kolben 2 zugfest miteinander verbunden. Diese Ausführung hat den Vorteil, daß der Kolben nicht nur zum Austreiben der Masse aus dem Spritzenkörper vorgeschoben, sondern auch zurückgezogen werden kann. Dies ist beispielsweise dann wichtig, wenn der Benutzer feststellt, daß er mehr von der kostspieligen Masse aus dem Spritzenkörper herausgedrückt hat, als es für den jeweiligen Anwendungsfall erforderlich ist. Der Überschuß kann durch Zurückziehen des Kolbens in den Spritzenkörper zurückgesaugt werden, um für den folgenden Anwendungsfall erhalten zu bleiben.

Das Farbmuster kann mit der Spindel verklebt oder verschweißt sein. Es kann auch zusätzlich oder statt dessen eine formschlüssige Verbindung vorgesehen sein, in die das Farbmuster (vorzugsweise unlösbar) eingeschnappt, eingeklemmt oder eingeklinkt wird.

## Patentansprüche

1. Vorratsspritze für Dentalmasse, insbesondere für Füllungen und Verblendungen, deren Kolben (2) durch eine Schraubspindel (5) vorschiebbar ist, die mittels einer Handhabe verdrehbar ist, dadurch gekennzeichnet, daß die Handhabe ein Farbmuster (8) für die in der Vorratsspritze enthaltene Dentalmasse (3) umfaßt.

2. Vorratsspritze nach Anspruch 1, dadurch gekennzeichnet, daß das schmal und flach ausgebildete Farbmuster (8) sich an das freie Ende der Spindel (5) etwa in deren Längsrichtung anschließt.

3. Vorratsspritze nach Anspruch 1 oder 2, dadurch gekennzeichnet, daß der Spritzenkörper (1) verengungsfrei mündet.

4. Vorratsspritze nach einem der Ansprüche 1 bis 3, dadurch gekennzeichnet, daß weder der Spritzenkörper noch die Spindel mit einer seitlich ausladenden Handhabe versehen sind.
